Europäisches Patentamt

European Patent Office

Office européen des brevets

(19)

(11) Numéro de publication: **0 270 806**
**A1**

(12) ## DEMANDE DE BREVET EUROPEEN

(21) Numéro de dépôt: **87115788.9**

(22) Date de dépôt: **27.10.87**

(51) Int. Cl.⁴ **G01N 33/04** , G01N 35/00

(30) Priorité: **30.10.86 FR 8615160**

(43) Date de publication de la demande:
**15.06.88 Bulletin 88/24**

(84) Etats contractants désignés:
**AT BE CH DE ES GB IT LI NL SE**

(71) Demandeur: **SOCIETE CIVILE DE BREVETS J.L.S.**
**La Mayrie**
**F-38770 La Motte d'Aveillans(FR)**

(72) Inventeur: **Savoyet, Jean-Louis**
**La Mayrie**
**F-38770 La Motte D'Aveillans(FR)**

(74) Mandataire: **Casalonga, Axel et al**
**BUREAU D.A. CASALONGA - JOSSE**
**Morassistrasse 8**
**D-8000 Munich 5(DE)**

(54) **Procédé et dispositif d'analyse spectrometrique automatique d'un liquide, notamment du lait.**

(57) Procédé et dispositif d'analyse spectrométrique d'un liquide, notamment du lait, caractérisé par le fait qu'il comprend divers postes (1, 2, 3, 4) assurant successivement le dosage du volume précis de liquide et son dépôt sur une plaquette (7), l'étalement de ce volume suivant une surface déterminée, de préférence combiné avec le séchage de la tache obtenue, l'analyse spectrométrique de la plaquette (7) munie de sa tache sèche, et un dispositif d'essuyage à l'aide d'une bande de papier-filtre (48) avec des détergents et solvants appropriés, un dispositif de déplacement (8,9,10), de préférence horizontal et rectiligne, assurant le déplacement d'une plaquette-support (5) portant au moins une plaquette (7) pour lui faire occuper successivement les divers postes.

FIG.1

EP 0 270 806 A1

## PROCEDE ET DISPOSITIF D'ANALYSE SPECTROMETRIQUE AUTOMATIQUE D'UN LIQUIDE, NOTAMMENT DU LAIT

L'invention concerne l'analyse d'un liquide, et plus particulièrement d'un liquide contenant des matières solides en suspension ou en solution dans un solvant, comme c'est le cas par exemple du lait, afin de déterminer les concentrations des divers constituants dans le solvant, lequel est naturellement de l'eau dans le cas du lait.

Pour réaliser cette analyse, l'un des procédés les plus connus est la spectrométrie infrarouge qui utilise des spectromètres constitués essentiellement :

-d'une source de rayonnement infrarouge munie de filtres de sélection de longueurs d'onde ou d'un dispositif monochromateur permettant le balayage dans un large spectre,

-d'au moins une cuve de confinement de l'échantillon analysé, cette cuve comprenant notamment deux parois parallèles traversées par le rayonnement,

-et d'un capteur permettant de détecter l'intensité du rayonnement ayant traversé la cuve et l'échantillon de faible épaisseur contenu entre les deux parois parallèles, afin de déterminer l'atténuation du rayonnement produite par cet échantillon.

La mesure est toujours comparative et utilise, soit un spectromètre à deux cuves, l'une contenant le solvant pur pour servir de référence et l'autre l'échantillon, le signal correspondant aux deux cuves étant envoyé alternativement sur le détecteur d'une manière synchrone, soit un spectromètre à cuve unique dans lequel deux faisceaux infarouges correspondant respectivement à la longueur d'onde d'analyse d'un constituant fortement absorbée par ce constituant, et à une longueur d'onde de référence absorbée de manière négligeable par ce constituant, sont envoyés alternativement sur le détecteur à travers le même échantillon également d'une manière synchrone.

Dans les deux cas, on utilise plusieurs longueurs d'onde caractéristiques correspondant au moins à chacun des constituants recherchés, c'est-à-dire dans le cas du lait, aux matières grasses, aux protéines et au lactose.

Ce procédé habituel d'analyse présente de nombreux inconvénients, dont le plus important est lié à la très faible épaisseur de l'échantillon, nécessaire pour atténuer le moins possible le signal traversant la cellule. Il en résulte que la cellule de mesure s'obstrue partiellement sous l'action d'une sédimentation des matières solides contenues dans le solvant lorsque plusieurs analyses successives sont effectuées sans nettoyage de la cellule. Par ailleurs, il est extrêmement difficile d'assurer de manière efficace ce nettoyage des faces intérieures extrêmement rapprochées de la cellule, de sorte que la persistance inévitable de traces résiduelles provenant, soit du produit analysé précédemment, soit du liquide de nettoyage, rend nécessaire un ré-étalonnage fréquent de l'appareil.

Les difficultés de manipulations, de mesures, de nettoyages et de ré-étalonnages, entre autres, nécessitent obligatoirement une utilisation en laboratoire des spectromètres connus et en interdisent pratiquement l'utilisation sur le terrain. De là, résulte un autre inconvénient qui est le délai important qui s'écoule entre un prélèvement d'échantillon et l'exécution de l'analyse, délai qui fausse par conséquent cette analyse lorsqu'il s'agit d'un produit altérable comme le lait, dans lequel l'acide lactique produit par dégradation microbienne fausse de manière importante la mesure. D'autre part, un délai encore plus important s'écoule entre tout prélèvement d'échantillon et la connaissance du résultat de l'analyse, ce qui est également un inconvénient important lorsque ce résultat est attendu en vue de la conduite du troupeau. Dans l'idéal, il serait donc nécessaire de pouvoir faire cette analyse d'une manière rapide automatique et sur le site.

Un autre inconvénient des méthodes usuelles est la présence du solvant, qui constiue presque toujours l'élément essentiel dans la composition du liquide et qui présente une absorption importante du rayonnement infrarouge. C'est le cas notamment de l'eau, solvant dans le cas du lait, dont la teneur en matiere sèche est toujours relativement faible par rapport à la teneur en eau. C'est ce phénomène qui pratiquement conduit à limiter l'épaisseur de l'échantillon à quelques microns, d'où les difficultés exposées plus haut. Il en résulte en outre une imprécision dans les mesures où l'absorption caractéristique des matières solides analysées ne représente qu'une faible valeur relative par rapport à l'absorption du solvant.

Le but de l'invention est de réaliser un procédé et un dispositif d'analyse spectrométrique qui éliminent les inconvénients précédents, en particulier les difficultés de manipulation, de délai et d'imprécision liées entre autres à la cuve et au solvant.

Le procédé selon l'invention consiste :

-à supprimer la cuve en déposant un petit volume de liquide parfaitement calibré sur une surface plane et horizontale d'un matériau transparent aux longueurs d'onde utilisées, puis en étalent ledit volume calibré sous la forme d'une tache de surface bien déterminée, par exemple circulaire, à l'aide d'un dispositif d'étalement, de préférence rotatif, ce qui assure finalement une épaisseur déterminée de l'échantillon, permettant ainsi au rayonement infrarouge de ne traverser qu'une paroi solide, mais en permettant surtout un nettoyage facile et efficace de cette surface plane par essuyage et polissage,

-à éliminer le solvant par séchage de cette tache, ce qui précisément est possible grâce au fait que la tache étalée offre une grande surface à l'air libre, et aussi grâce à la grande facilité de nettoyage ultérieur de la tache séchée.

Le dispositif pour la mise en oeuvre du procédé selon l'invention comporte essentiellement un poste de production d'un volume calibré de liquide et son dépôt sur un porte-échantillon, un dispositif rotatif d'étalement de la tache, de préférence combiné avec son séchage à l'air chaud, un dispositif d'analyse spectrométrique de la tache séchée sur le porte-échantillon, un dispositif de nettoyage et d'essuyage de la tache, enfin un moyen mécanique déplaçant automatiquement le porte-échantillon pour lui faire occuper successivement dans le temps les divers postes précédents, de préférence par un mouvement rectiligne et horizontal. Toutefois, sans s'écarter du procédé décrit, à l'utilisation d'un dispositif linéaire pourrait se substituer celle d'un dispositif circulaire qui conduirait à un résultat semblable.

D'autres particularités de l'invention apparaîtront dans la description qui va suivre d'un mode de réalisation pris comme exemple et représenté sur le dessin annexé, sur lequel :

la figure 1 est une vue en élévation avec coupe partielle de l'ensemble du dispositif, et

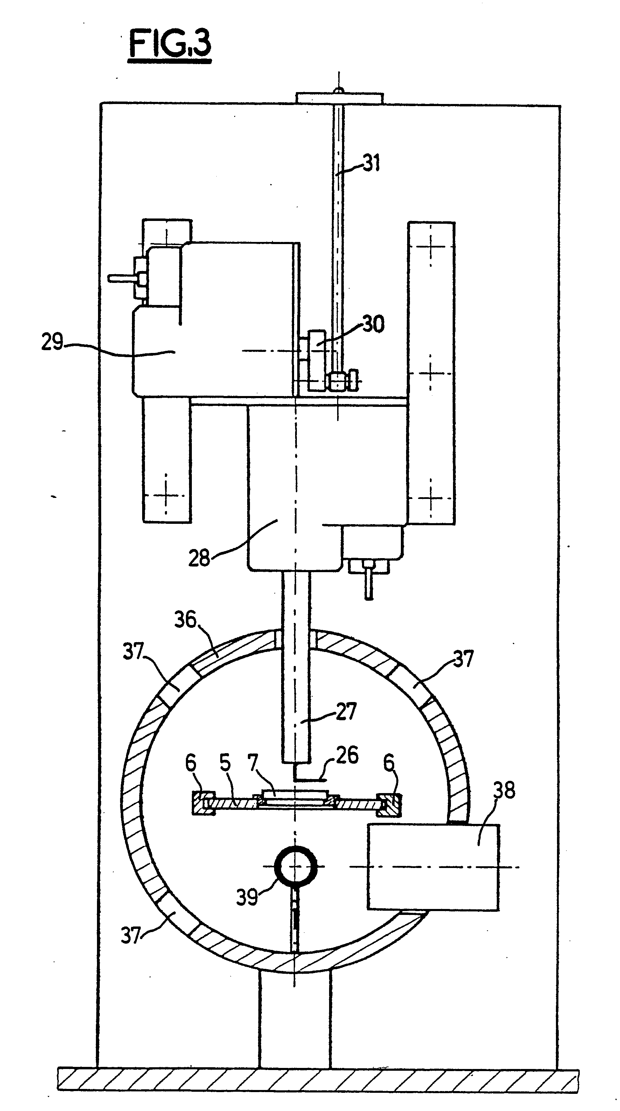

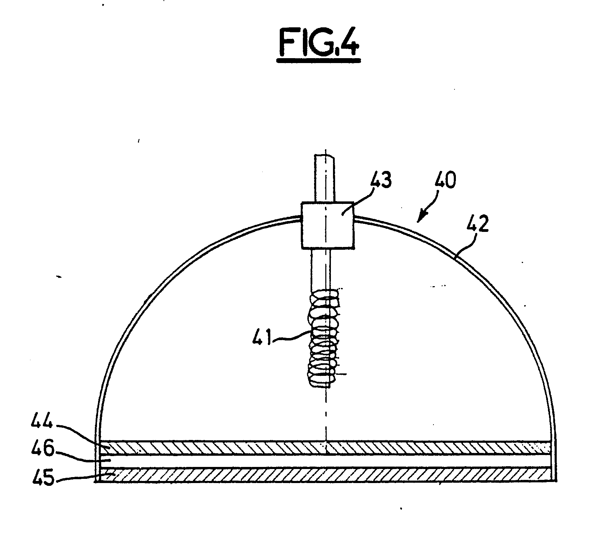

les figures 2, 3, 4 et 5 sont respectivement des coupes verticales transversales selon II-II, III-III, IV-IV et V-V de la figure 1.

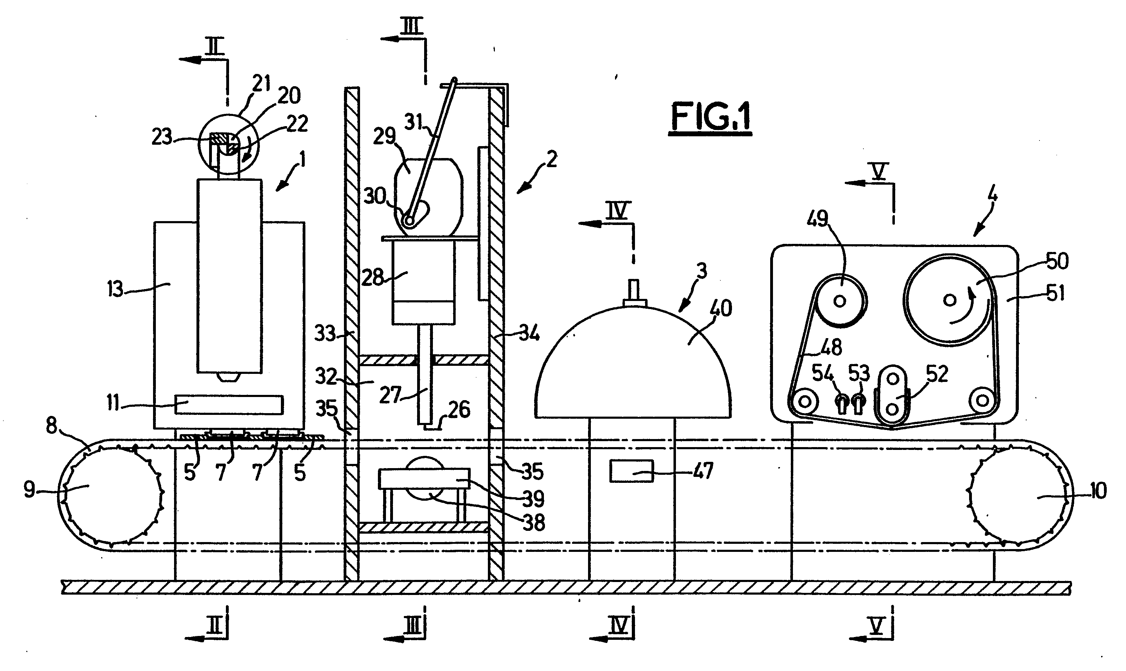

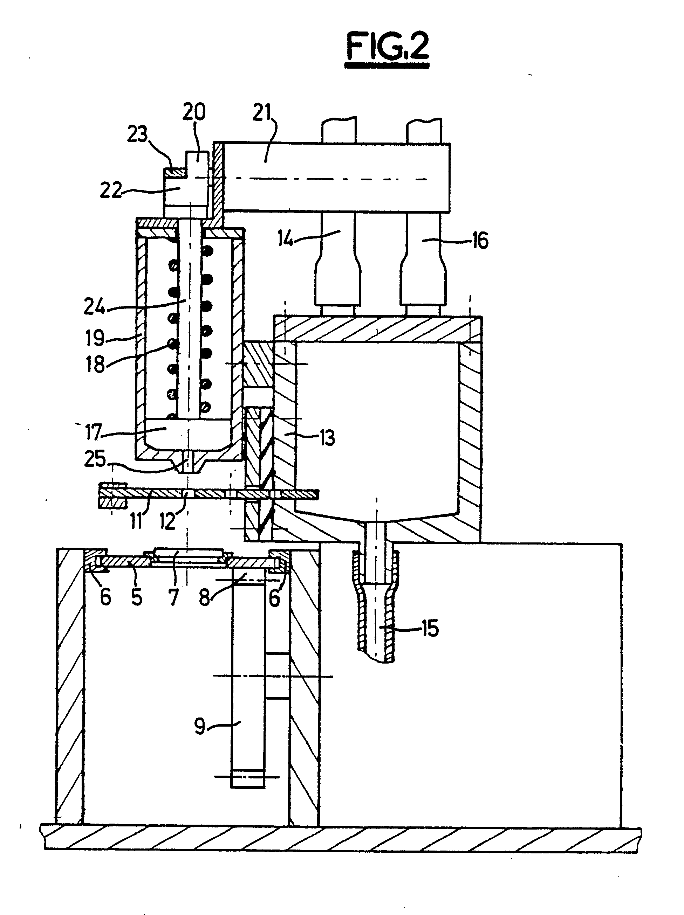

On voit sur la figure 1 les divers postes 1, 2, 3 et 4 par lesquels passe successivement un porte-échantillon unique 5 à la faveur d'un déplacement horizontal, de préférence rectiligne. Ce porte-échantillon 5 présente la forme d'une plaque coulissant entre deux guides latéraux 6, visibles sur la figure 2 et les suivantes, et il porte une, ou de préférence deux plaquettes 7 à faces parallèles faites d'un matériau transparent aux longueurs d'onde utilisées. En particulier, ce matériau peut être du silicium ou du germanium.

En variante, le porte-échantillon peut être suivi d'un ou plusieurs autres semblables permettant une réduction du temps d'analyse, c'est-à-dire que pendant une opération de séquence, une autre peut se réaliser (ex : premier porte-échantillon analyse, un second porte-échantillon étalement, séchage, etc.).

De même, le nombre de pastilles peut être plus important pour chaque séquence afin de réaliser une double analyse permettant un moyennage, une augmentation de la précision, une détection d'aberration par comparaison des niveaux respectifs obtenus.

Dans le mode de réalisation représenté, le moyen de déplacement rectiligne de la plaque porte-échantillon 5 dans ses guides 6 est assuré par une courroie crantée 8 dont elle est solidaire et qui est renvoyée entre deux poulies d'extrémité 9 et 10, dont l'une est entraînée par un moteur pas à pas non représenté. Ce moteur est naturellement à deux sens de marche et permet après chaque opération de déplacer la plaque porte-échantillon 5 dans la direction voulue et de la distance précise voulue, afin de passer successivement par les divers postes 1, 2, 3 et 4 dans l'ordre indiqué, sans qu'il soit nécessaire pour cela que les quatre postes soient distribués régulièrement dans l'espace selon ce même ordre.

Tout autre mode de guidage est envisageable (ex : plateau tournant commandé par moteur pas à pas ou dispositif tachymétrique).

Le premier poste 1 est un poste de dosage dont le but est de prélever un volume faible mais précis du liquide à analyser et le déposer sur une plaquette 7. Pour cela, on peut utiliser n'importe quel dispositif de dosage volumétrique précis indépendant de la tension superficielle du liquide qui peut varier. Dans l'exemple choisi, ce dispositif est constitué par une plaque coulissante 11, visible en particulier sur la figure 2, cette plaque, de préférence en céramique, ayant des faces parallèles rectifiées à une cote d'épaisseur bien précise, tout en étant percée d'au moins un alésage 12, également d'un diamètre précis, dont les parois intérieures sont revêtues d'un produit non mouillant tel que du polytétrafluoréthylène. Par ailleurs, cette plaque 11 peut coulisser de manière étanche entre des garnitures pour pénétrer à travers la paroi 13 d'un réservoir d'échantillon dans lequel l'échantillon à analyser est amené par un tuyau 14, puis évacué après analyse par un tuyau 15. Lorsque le dispositif de prélèvement d'échantillon fonctionne par dépression, on peut en outre prévoir un tuyau supplémentaire 16 à la partie supérieure pour fournir la dépression.

Un mécanisme de déplacement en va-et-vient non représenté produit le coulissement de la plaque 11 depuis une position complètement enfoncée, où son trou 12 se trouve complètement immergé dans le liquide, jusqu'à une position complètement sortie où il se trouve juste à l'aplomb d'une plaque 7 comme représenté sur la figure 2. Dans cette position, le volume cylindrique extrêmement précis de liquide qui a été ainsi calibré se trouve généralement retenu par capillarité, sans que pour autant les forces de tension capillaires puissent intervenir dans la détermination de ce volume, comme cela serait le cas par exemple avec un compte-gouttes.

Pour chasser ce volume et le faire tomber sur la plaquette 7, on utilise un simple jet d'air, réalisé dans l'exemple à l'aide d'un piston 17 chargé par un ressort 18 et se déplaçant dans un cylindre 19, grâce à une came 20 entraînée par un moteur 21. Cette came 20 comporte un doigt 22 susceptible rencontrer et de soulever un talon 23 solidaire de la tige 24 du piston 17, afin de le soulever en comprimant le ressort 18. Lorsque le doigt 22 échappe latéralement du talon 23, la chute brusque du piston chasse un petit volume d'air par l'orifice 25 qui souffle dans l'axe du trou 12 et chasse ainsi le volume de liquide calibré qui tombe sur la plaquette 7, formant sur celle-ci un ménisque. Un autre moyen tel générateur d'air ou de gaz peut être employé.

La plaque 30 d'échantillon 5 est alors déplacée pour être amenée au poste 2 où se produit l'étalement et le séchage. Cet étalement est produit à l'aide d'un fil coudé 26 en tungstène ou en produit non mouillant, ce fil étant porté par une broche 27 pour être entraîné en rotation par un moteur 28, après avoir été descendu au contact de la plaquette 7 par un mécanisme approprié, constitué par exemple par un moteur 29, une manivelle 30 et une bielle 31.

La rapide rotation du fil coudé 26 au contact de la plaquette 7 produit l'étalement du volume précis de liquide déposé sous la forme d'une tache circulaire d'un diamètre bien précis et d'une façon répétitive. En outre, elle assure une excellente homogénéisation du produit, par rupture des grosses molécules de matière grasse jointe à une excellente dispersion.

L'opération se pratique de préférence à l'intérieur de l'enceinte 32 d'un four constitué par des parois parallèles 33 et 34 munies de fenêtres 35 pour l'entrée et la sortie de la plaque 5, et une paroi 36, par exemple cylindrique, munie d'orifices 37 pour la circulation de l'air, qui est soufflé par un ventilateur 38 sur une résistance chauffante 39 placée de préférence au dessous du porte-échantillon pour chauffer à la fois la plaquette et l'air. En variante, tout autre mode de chauffage pourrait être utilisé, tel qu'un chauffage à rayonnement infrarouge. En outre, le chauffage du four est de préférence thermostaté. Ce chauffage doit être suffisant pour sécher rapidement le faible volume de liquide étalé sur la surface circulaire indiquée, mais en même temps insuffisante pour altérer le produit.

Après séchage et relevage de la broche 27 par le mécanisme de soulèvement 29, 30, 31, la plaquette 7, ainsi munie d'une tache sèche du produit à analyser, débarrassé de son solvant et ne représentant par conséquent que les matières sèches contenues dans le volume initial précis réparti sur la surface circulaire précise indiquée, se trouve déplacée à nouveau pour l'amener au troisième poste 3 où se produit l'analyse spectrométrique proprement dite, par exemple à l'aide d'un appareillage usuel comprenant une source de rayonnement infrarouge 40 et des filtres, ou encore un monochromateur tel que décrit dans le brevet français 2 540 626, et naturellement un capteur de rayonnement 47.

A titre de perfectionnement, l'invention prévoit d'utiliser comme source de rayonnement infrarouge 40 un filament de carbone 41 disposé dans une paroi hémisphérique ou parabolique 42 formant réflecteur, de forme telle qu'assurant une excellente focalisation dans le cas considéré comme couvrant la plus grande partie de la surface de l'extrait sec et reçu pour sa totalité par le ou les capteurs, avec de préférence un dispositif Peltier 43 assurant le refroidissement de ce réflecteur. La face inférieure du réflecteur 42 est fermée par un disque 44 en matériau transparent aux infrarouges, par exemple en silicum, et de préférence également par un deuxième disque semblable 45 séparé du précédent par un vide 46 total ou partiel, ceci afin d'éviter les ré-émissions infrarouges parasites. Le vide est aussi réalisé dans l'enceinte.

En dessous du niveau du porte-échantillon 5, se trouve placé le détecteur 47 d'un type approprié. En particulier, on peut utiliser un substrat sur lequel sont réalisés ou fixés un ou plusieurs phototransistors sensibles chacun à une bande de fréquence bien déterminée, ou recouverts chacun d'un filtre approprié laissnt passer la bande voulue. Cette disposition permet de réaliser simultanément les mesures pour les diverses bandes, ou tout au moins d'éviter la rotation des filtres ou du monochromateur, le principe de l'invention restant applicable même en cas de rotation des filtres.

Dans le cas plus particulier du lait, les bandes à utiliser sont de préférence les suivantes :

a) la bande de 2990 à 2880 ondes/cm, soit de 3,355 à 3,472 $\mu$;

b) la bande de 1770 à 1720 ondes/cm, soit de 5,650 à 5,814 $\mu$;

c) la bande de 1580 à 1490 ondes/cm, soit de 6,329 à 6,711 $\mu$;

d) la bande de 1070 à 1000 ondes/cm, soit de 9,345 à 10 $\mu$.

La bande a), qui est la plus couramment utilisée, est sensible aux matières grasses du lait, mais également partiellement sensible aux protéines. Au contraire, la bande b) est sensible à la teneur en matières grasses et pratiquement pas aux protéines ni au lactose, de sorte qu'elle sera utilisée de préférence. Les bandes c) et d) sont sensibles respectivement aux teneurs en protéines et en lactoses. On utilisera donc les quatre bandes indiquées, ou au moins les trois dernières.

Comme pour les systèmes à cuve, on peut utiliser une bande de longueurs d'onde supplémentaire, pratiquement pas absorbée par les produits à analyser et qui sert de référence pour la mesure, ou encore, par anologie avec le dispositif à deux cuves, utiliser une deuxième plaquette 7 qui ne reçoit aucun liquide et qui sert de comparaison en utilisant les rayonnements caractéristiques indiqués, ce procédé permettant aussi de connaître l'état de vieillissement ou autre altération (oxydation, etc.) résultant de l'usage :

a) de la source,

b) de la plaquette recueillant le lait, ce par comparaison du niveau de signal entre la plaquette de référence et les autres.

La comparaison des diverses intensités de rayonnement ainsi captées par le détecteur 47 permet de la sorte de déterminer les teneurs recherchées avec une grande précision, en raison de l'absence de cuve, de l'absence d'eau, de la mesure comparative précise, et enfin de la grande facilité de nettoyage de la surface supérieure de la ou des plaquette(s) 7 avant toute mesure.

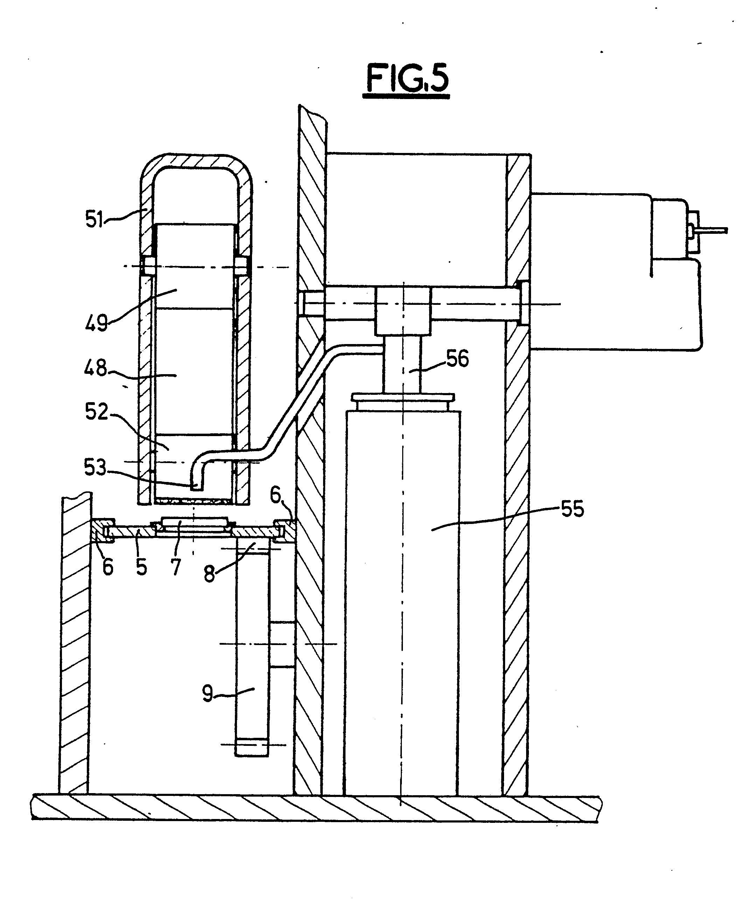

Ce nettoyage est effectué automatiquement au peste 4 à l'aide d'un ruban de papier-filtre 48 monté entre une bobine débitrice 49 et une bobine réceptrice 50 d'une cassette 51, avec un tampon presseur 52 appuyant cette bande sur le dessus d'une plaquette en cours de nettoyage, deux ajutages 53 et 54 étant en outre prévus pour distribuer un liquide détergent et un produit de rinçage à partir de réservoirs appropriés tels que 55 sur la figure 5 et à l'aide d'un dispositif d'injection 56 entraîné par un moteur approprié.

Pour assurer ce nettoyage, on produit donc le déplacement de la plaquette par le moteur pas-à-pas indiqué plus haut jusqu'à amener la plaquette 7 sous le presseur 52, sous une zone du papier-filtre 48 préalablement mouillée de détergent par l'ajutage 53. Après abaissement du presseur 52, le même moteur pas-à-pas produit un mouvement de va-et-vient longitudinal du porte-échantillon pour produire le déplacement horizontal relatif assurant le nettoyage superficiel de la plaquette. Après distribution du produit de rinçage par l'ajutage 54 et l'avancement d'une longueur appropriée de papier-filtre 48, on produit de la même façon le rinçage de la plaquette 7, et enfin l'essuyage à l'aide d'une zone sèche de papier-filtre. Un effet semblable est obtenu (nettoyage) si la bande de papier est animée d'un mouvement alternatif, la conjugaison des deux possibilités pouvant être utilisée.

La deuxième plaquette 7 servant de comparaison n'a pas à être nettoyée à chaque cycle, mais peut naturellement l'être automatiquement de la même façon chaque fois qu'il est nécessaire.

L'appareil selon l'invention peut ainsi fonctionner d'une manière précise, sûre et absolument automatique, sans nécessiter aucune manipulation délicate ni aucun ré-étalonnage. En outre, il peut facilement être réalisé sous une forme compacte et transportable permettant son utilisation sur le site, en particulier sur le lieu de la traite, avec tous les avantages envisagés plus haut, consécutifs à l'absence d'altération du produit et à l'obtention de résultats rapides facilitant la conduite du troupeau.

Dans les exemples décrits, le produit principal présenté comme pouvant être objet de l'analyse est le lait. Afin d'analyser certains laits présentant de très fortes concentrations (ex : lait de bufflonne 130 g de matière grasse/kilo), le volume calibré nécessaire à un bon étalement peut être réduit etadditionné d'eau déminéralisée.

Se trouve ainsi réalisée une dilution qui ne diminue en rien la mesure, compte tenu de l'opération de séchage, l'eau dans le cas présenté ne servant qu'à un meilleur étalement.

Il est à souligner l'absence de nécessité de réalisation d'un volum calibré de grande précision de l'eau introduite.

Dans le même esprit, divers corps solubles ou rendus solubles par différents moyens tels chauffage, fragmentation préalable, etc., peuvent dans un premier temps être dissouts ou intimement associés dans un milieu solvant ou liant; dans le cas ci-dessus, eau. Le solvant ou liant extrait sans que sa présence à un quelconque moment n'ait modifié autrement que de manière connue, exploitable le produit principal, celui-ci au départ pouvant présenter divers états, poudre, pâte, liquide (ex : fromage de toute nature, poudre de poisson, etc.), les moyens décrits utilisables afin de déterminer les concentrations recherchées, la gamme de longueurs d'onde infrarouge utilisée variant en fonction de la nature du produit recherché.

**Revendications**

1. Procédé d'analyse spectrométrique d'un liquide, notamment du lait, par mesure de l'absorption dans diverses bandes de fréquences d'un rayonnement infrarouge, caractérisé par le fait que l'on réalise successivement les opérations suivantes :

-on produit le dosage précis d'un petit volume de liquide à analyser que l'on dépose sur la surface supérieure plane d'une plaquette (7) d'un produit transparent au rayonnement utilisé,

-on étale ce volume de liquide sur la plaquette selon une surface bien déterminée, de préférence circulaire et à l'aide d'un dispositif rotatif,

-on sèche la tache liquide ainsi obtenue, de préférence en même temps que se produit son étalement, jusqu'à l'obtention d'une tache sèche,
-on soumet la plaquette (7) munie de sa tache sèche à l'analyse spectrométrique en l'absence du solvant du liquide, et
-on nettoie la surface supérieure de la plaquette par essuyage et polissage avant la mesure suivante.

2. Dispositif pour la mise en oeuvre du procédé selon la revendication 1, caractérisé par le fait qu'il comprend divers postes (1, 2, 3, 4) assurant successivement le dosage du volume précis de liquide et son dépôt sur une plaquette (7), l'étalement de ce volume suivant une surface déterminée, de préférence combiné avec le séchage de la tache obtenue, l'analyse spectrométrique de la plaquette (7) munie de sa tache sèche, et un dispositif d'essuyage à l'aide d'une bande de papier-filtre (48) avec des détergents et solvants appropriés, un dispositif de déplacement (8,9,10) de préférence horizontal et rectiligne, assurant le déplacement d'une plaquette-support (5) portant au moins une plaquette (7) pour lui faire occuper successivement les divers postes.

3. Dispositif suivant la revendication 2, caractérisé par le fait que le dispositif de dosage du petit volume précis de liquide est constitué par une plaque (11) munie d'un orifice (12) à paroi non mouillable et coulissant de manière étanche à travers la paroi d'une cuve (13) contenant le produit à analyser, de manière que l'orifice puisse être immergé dans le liquide pour extraire le volume cylindrique précis de liquide vers l'extérieur au-dessus de la plaquette (7), ce volume étant ensuite chassé sur cette plaquette par un bref jet d'air.

4. Dispositif selon la revendication 3, caractérisé par le fait que ladite plaque coulissante (11) est réalisée en céramique et que l'orifice de calibrage (12) est revêtu intérieurement d'un produit non mouillant.

5. Dispositif selon une des revendications 2 à 4, caractérisé par le fait que le dispositif d'étalement est constitué par un fil coudé (26) monté à l'extrémité d'une broche rotative (27) munie d'un moyen d'approche et d'éloignement (29-30-31) pour venir en contact avec la plaquette (7), l'ensemble étant contenu dans une enceinte (32) munie d'un moyen de chauffage (39) et de ventilation (38).

6. Dispositif selon une des revendications 2 à 5, caractérisé par le fait que la source de rayonnement infrarouge (40) est constituée par un filament de carbone (41) contenu dans un réflecteur (42) muni d'un refroidisseur Peltier (43) et fermée par deux disques (44, 45) en matériau transparent aux infrarouges, séparés par le vide.

7. Dispositif selon une des revendications 2 à 6, caractérisé par le fait que le détecteur de rayonnement (47) est formé par un réseau de détecteurs individuels montés sur un même substrat et détectant chacun une des bandes de fréquences utilisées pour la mesure.

8. Dispositif selon une des revendications 2 à 7, caractérisé par le fait que les bandes de fréquences utilisées pour la mesure comprennent au moins les trois bandes suivantes :
-1770 à 1720 ondes/cm (5,650 à 5,814 $\mu$),
-1580 à 1490 ondes/cm (6,329 à 6,711 $\mu$), et
-1070 à 1000 ondes/cm (9,345 à 10 $\mu$).

9. Dispositif selon une des revendications 2 à 8, caractérisé par le fait que le mouvement relatif horizontal, assurant les effets de nettoyage par essuyage et polissage de la plaquette (7) au poste de nettoyage, est produit par le même dispositif assurant la translation de la plaquette porte-échantillon (5) aux divers postes, à la faveur d'une alternance des deux sens de déplacement.

10. Dispositif selon une des revendications 2 à 9, caractérisé par le fait que la bande de papier-filtre (48) servant au nettoyage est chargée à l'aide d'une cassette (51) contenant à la fois la bobine débitrice (49) et réceptrice (50).

FIG.1

FIG.2

## FIG.3

# FIG.4

# FIG.5

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | NL-A-7 201 571 (BECKER DELFT N.V.) * Page 6, lignes 1-6; figure * | 1 | G 01 N  33/04 G 01 N  35/00 |
| A | FR-A-2 161 618 (TECHNICON INSTRUMENTS) * Figure 1; page 10, lignes 1-19 * | 1 | |
| A | FR-A-2 153 083 (VICKERS LTD) * Figure 1; page 2, ligne 21 - page 3, ligne 7 * | 1,5 | |
| A | US-A-4 310 763 (SHIELDS) | | |
| A | US-A-4 257 862 (SCHNIPELSKY) | | |

DOMAINES TECHNIQUES RECHERCHES (Int. Cl.4)

G 01 N  33/00
G 01 N  35/00
G 01 N  21/00

Le présent rapport a été établi pour toutes les revendications

| Lieu de la recherche | Date d'achèvement de la recherche | Examinateur |
|---|---|---|
| LA HAYE | 24-02-1988 | KLEIKAMP B.M.H.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
......................................................................................
& : membre de la même famille, document correspondant

EPO FORM 1503 03.82 (P0402)